(19) Europäisches Patentamt
European Patent Office
Office européen des brevets

(11) Veröffentlichungsnummer : **0 090 993**
**B1**

(12) # EUROPÄISCHE PATENTSCHRIFT

(45) Veröffentlichungstag der Patentschrift : **07.05.86**

(21) Anmeldenummer : **83102754.5**

(22) Anmeldetag : **21.03.83**

(51) Int. Cl.⁴ : **C 07 D233/60**, C 07 D303/08, C 07 D303/22, C 07 D249/08, A 61 K 31/41, A 61 K 31/415

(54) **Substituierte Hydroxyalkyl-azole, Verfahren zu ihrer Herstellung und ihre Verwendung als Antimykotika.**

(30) Priorität : **02.04.82 DE 3212388**

(43) Veröffentlichungstag der Anmeldung : **12.10.83 Patentblatt 83/41**

(45) Bekanntmachung des Hinweises auf die Patenterteilung : **07.05.86 Patentblatt 86/19**

(84) Benannte Vertragsstaaten :
**AT BE CH DE FR GB IT LI LU NL SE**

(56) Entgegenhaltungen :
**EP-A- 0 029 542**
**DE-A- 2 736 122**

(73) Patentinhaber : **BAYER AG**
**Konzernverwaltung RP Patentabteilung**
**D-5090 Leverkusen 1 Bayerwerk (DE)**

(72) Erfinder : **Elbe, Hans-Ludwig, Dr.**
**Dasnoekel 59**
**D-5600 Wuppertal 11 (DE)**
Erfinder : **Büchel, Karl Heinz, Prof. Dr.**
**Dabringhausener Strasse 42**
**D-5093 Burscheid (DE)**
Erfinder : **Schaller, Klaus, Dr.**
**Am Sonnenschein 38**
**D-5600 Wuppertal 1 (DE)**
Erfinder : **Plempel, Manfred, Dr.**
**Zwengenberger Strasse 3c**
**D-5657 Haan (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 090 993 B1

Jouve, 18, rue St-Denis, 75001 Paris, France

**Beschreibung**

Die Erfindung betrifft neue substituierte Hydroxyalkyl-azole, ein Verfahren zu ihrer Herstellung und ihre Verwendung als Antimykotika.

Es ist bereits bekannt geworden, daß bestimmte Hydroxyalkylazolyl-Derivate, wie z. B. 1,2-Bis(4-chlorphenyl)-2-hydroxy-3-(imidazol-1-yl)-propan oder 1,1-Bis(3-chlorphenyl)-1-hydroxy-2-(imidazol-1-yl)-ethan und 2-(4-Biphenylyl)-2-hydroxy-1-phenyl-3-(1,2,4-triazol-1-yl)-propan, gute antimykotische Eigenschaften aufweisen [vergleiche DE-OS 26 23 129 und DE-OS 28 51 086]. Die Wirkung dieser Verbindungen ist jedoch, insbesondere bei niedrigen Anwendungskonzentrationen, nicht immer voll befriedrigend.

Es wurden neue substituierte Hydroxyalkyl-azole der allgemeinen Formel I

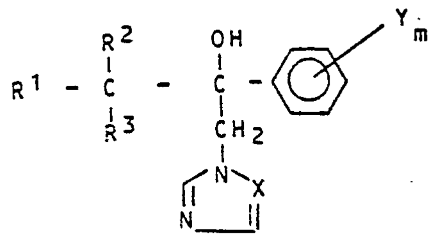

(I)

in welcher

$R^1$ für jeweils einfach bis zweifach, gleich oder verschieden substituiertes Phenyl, —O-Phenyl, —S-Phenyl, —SO-Phenyl, —$SO_2$-Phenyl, —$CH_2$-Phenyl, —$CH_2$—O-Phenyl, —$CH_2$—S-Phenyl, —$CH_2$—SO-Phenyl oder —$CH_2$—$SO_2$-Phenyl, wobei als Phenylsubstituenten die Bedeutungen von Y genannt seien ;

$R^2$ für gradkettiges oder verzweigtes Alkyl mit 1 bis 4 Kohlenstoffatomen ;

$R^3$ für gradkettiges oder verzweigtes Alkyl mit 1 bis 4 Kohlenstoffatomen ;

X für ein Stickstoffatom oder die CH-Gruppe ;

Y für Halogen, Alkyl mit 1 bis 4 Kohlenstoffatomen, Cycloalkyl mit 5 bis 7 Kohlenstoffatomen, Alkoxy und Alkylthio mit jeweils 1 bis 4 Kohlenstoffatomen, Halogenalkyl und Halogenalkoxy sowie Halogenalkylthio mit jeweils 1 bis 2 Kohlenstoff- und 1 bis 5 gleichen oder verschiedenen Halogenatomen sowie für jeweils gegebenenfalls durch Halogen und Alkyl mit 1 bis 2 Kohlenstoffatomen substituiertes Phenyl, Phenoxy, Phenylalkyl sowie Phenylalkoxy mit 1 bis 2 Kohlenstoffatomen im Alkylteil bzw. im Alkoxyteil ; und

m für die Zahlen 0, 1, 2 oder 3 stehen

sowie deren Säureadditionssalze gefunden, insbesondere diejenigen Verbindungen der allgemeinen Formel I, in denen

$R^1$ für jeweils einfach bis zweifach, gleich oder verschieden substituiertes Phenyl, —O-Phenyl, —S-Phenyl, —SO-Phenyl, —$SO_2$-Phenyl, —$CH_2$-Phenyl, —$CH_2$—O-Phenyl, —$CH_2$—S-Phenyl, —$CH_2$—SO-Phenyl oder —$CH_2$—$SO_2$-Phenyl steht, wobei als Phenylsubstituenten die Bedeutung von Y genannt seien ;

$R^2$ für Methyl oder Ethyl steht ;

$R^3$ für Methyl oder Ethyl steht ;

X für ein Stickstoffatom oder die CH-Gruppe steht ;

Y für Fluor, Chlor, Brom, Methyl, Isopropyl, tert.-Butyl, Cyclohexyl, Methoxy, Methylthio, Trifluormethyl, Trifluormethoxy, Trifluormethylthio sowie für gegebenenfalls durch Fluor, Chlor, Methyl substituiertes Phenyl, Phenoxy, Benzyl oder Benzyloxy steht ; und

m für die Zahlen 0, 1 oder 2 steht.

Die Verbindungen der allgemeinen Formel I besitzen ein asymmetrisches Kohlenstoffatom und können deshalb in den beiden optischen Isomerenformen anfallen.

Weiterhin wurde gefunden, daß man die substituierten Hydroxyalkyl-azole der allgemeinen Formel I erhält, wenn man Oxirane der allgemeinen Formel II

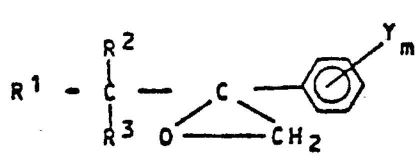

(II)

in welcher $R^1$, $R^2$, $R^3$, Y und m die oben angegebene Bedeutung haben, mit Azolen der allgemeinen Formel III

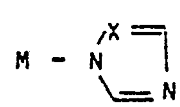

(III)

in welcher

X die oben angegebene Bedeutung hat und

M für Wasserstoff oder ein Alkalimetall steht,

in Gegenwart eines Verdünnungsmittels und gegebenenfalls in Gegenwart einer Base umsetzt, und gegebenenfalls die so erhaltenen Hydroxyalkyl-azole der allgemeinen Formel Ia

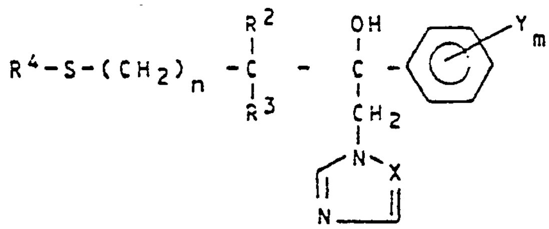

(Ia)

in welcher

$R^2$, $R^3$, X, Y und m die oben angegebene Bedeutung haben,

$R^4$ für gegebenenfalls durch Y substituiertes Phenyl steht und

n für die Zahlen 0 oder 1 steht,

nach bekannten Methoden in üblicher Weise oxidiert.

An die so erhaltenen Verbindungen der allgemeinen Formel I kann gegebenenfalls anschließend eine Säure addiert werden.

Die neuen substituierten Hydroxyalkyl-azole der allgemeinen Formel I weisen starke antimykotische Eigenschaften auf. Dabei zeigen überraschenderweise die erfindungsgemäßen Verbindungen insbesondere eine bessere, therapeutisch nutzbare in-vivo-Wirksamkeit als die aus dem Stand der Technik bekannten Verbindungen 1,2-Bis(4-chlorphenyl)-2-hydroxy-3-(imidazol-1-yl)-propan oder 1,1-Bis(3-chlorphenyl)-1-hydroxy-2-(imidazol-1-yl)-ethan und 2-(4-Biphenylyl)-2-hydroxy-1-phenyl-3-(1,2,4-triazol-1-yl)-propan, welche chemisch ähnliche Verbindungen sind. Die erfindungsgemäßen Stoffe stellen somit eine Bereicherung der Pharmazie dar.

Außerdem sind die neuen substituierten Hydroxyalkylazole interessante Zwischenprodukte. So können z. B. die Verbindungen der allgemeinen Formel I an der Hydroxygruppe in üblicher Weise in die entsprechenden Ether überführt werden. Weiterhin können durch Umsetzung mit z. B. Acylhalogeniden oder Carbamoylchloriden in prinzipiell bekannter Weiser Acyl- oder Carbamoyl-Derivate der Verbindungen der allgemeinen Formel I erhalten werden.

Erfindungsgemäße Verbindungen sind auch Additionsprodukte aus Säuren und denjenigen substituierten Hydroxyalkyl-azolen der allgemeinen Formel I, in denen die Substituenten $R^1$, $R^2$, $R^3$, X und $Y_m$ die Bedeutungen haben, die bereits vorzugsweise für diese Substituenten genannt wurden.

Zu den Säuren, die addiert werden können, gehören vorzugsweise Halogenwasserstoffsäuren, wie z. B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z. B. Essigsäure, Maleinsäure, Bernsteinsäure, Fumarsäure, Weinsäure, Citronensäure, Salicylsäure, Sorbinsäure und Milchsäure, sowie Sulfonsäuren, wie p-Toluolsulfonsäure und 1,5-Naphthalindisulfonsäure.

Im einzelnen seien außer den bei den Herstellungsbeispielen genannten Verbindungen die folgenden Verbindungen der allgemeinen Formel I genannt (X steht sowohl für ein Stickstoffatom als auch die CH-Gruppe) :

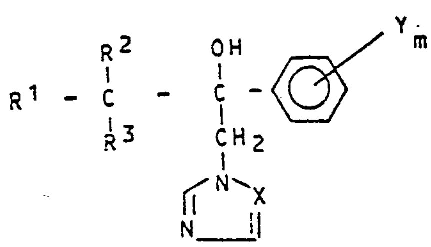

(I)

| $R^1$ | $R^2$ | $R^3$ | $Y_m$ |
|---|---|---|---|
| 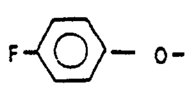 | $CH_3$ | $CH_3$ | 2-Cl |
| 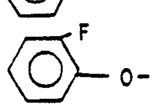 | $CH_3$ | $CH_3$ | 2-Cl |

(Fortsetzung)

| $R^1$ | $R^2$ | $R^3$ | $Y_m$ |
|---|---|---|---|
| 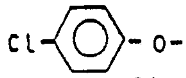 | $CH_3$ | $CH_3$ | 3-Cl |
| 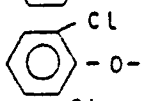 | $CH_3$ | $CH_3$ | 3,4-$Cl_2$ |
| 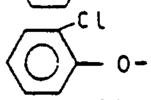 | $CH_3$ | $CH_3$ | 3-Cl |
| 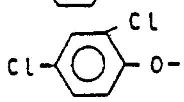 | $CH_3$ | $CH_3$ | 3-Cl |
| 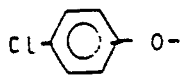 | $CH_3$ | $CH_3$ | 2,4-$Cl_2$ |
| 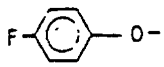 | $CH_3$ | $CH_3$ | 2,4-$Cl_2$ |
| 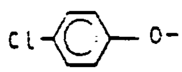 | $CH_3$ | $CH_3$ | 3,4-$Cl_2$ |
| 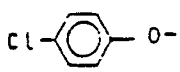 | $CH_3$ | $CH_3$ | 2-$CH_3$,4-Cl |
| 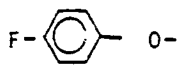 | $CH_3$ | $CH_3$ | 3,4-$Cl_2$ |
| 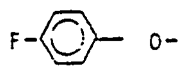 | $CH_3$ | $CH_3$ | 2-$CH_3$,4-Cl |
| 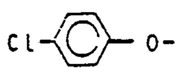 | $CH_3$ | $CH_3$ | 2-Cl |
| 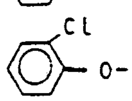 | $CH_3$ | $CH_3$ | 2-Cl |
| 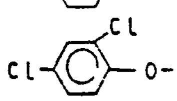 | $CH_3$ | $CH_3$ | 4-Cl |
| 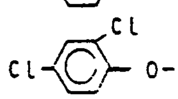 | $CH_3$ | $CH_3$ | 2,4-$Cl_2$ |
| 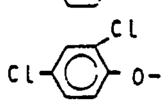 | $CH_3$ | $CH_3$ | 2-Cl |
| 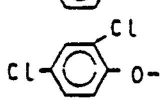 | $CH_3$ | $CH_3$ | 4-F |
| 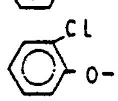 | $CH_3$ | $CH_3$ | 4-F |
| 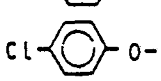 | $CH_3$ | $CH_3$ | 4-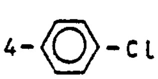 |
| 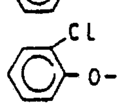 | $CH_3$ | $CH_3$ | 4-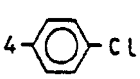 |
| 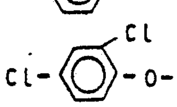 | $CH_3$ | $CH_3$ | 4-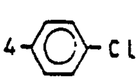 |

(Fortsetzung)

| $R^1$ | $R^2$ | $R^3$ | $Y_m$ |
|---|---|---|---|
| 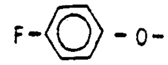 | $CH_3$ | $CH_3$ | 4-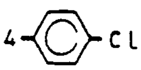 |
| 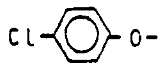 | $CH_3$ | $CH_3$ | 4-O-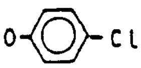 |
| 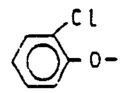 | $CH_3$ | $CH_3$ | 4-Cl |
| 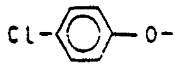 | $CH_3$ | $CH_3$ | 2-F |
| 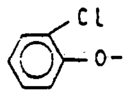 | $CH_3$ | $CH_3$ | 2-F |
| 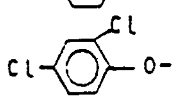 | $CH_3$ | $CH_3$ | 2-F |
| 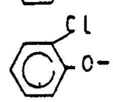 | $CH_3$ | $CH_3$ | 2,4-$Cl_2$ |
| 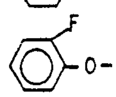 | $CH_3$ | $CH_3$ | 2,4-$Cl_2$ |
| 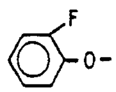 | $CH_3$ | $CH_3$ | 4-Cl |
| 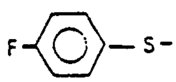 | $CH_3$ | $CH_3$ | 2-Cl |
| 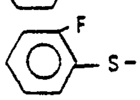 | $CH_3$ | $CH_3$ | 2-Cl |
| 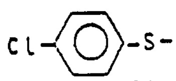 | $CH_3$ | $CH_3$ | 3-Cl |
| 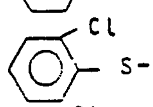 | $CH_3$ | $CH_3$ | 3,4-$Cl_2$ |
| 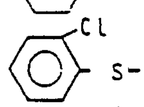 | $CH_3$ | $CH_3$ | 3-Cl |
| 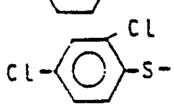 | $CH_3$ | $CH_3$ | 3-Cl |
| 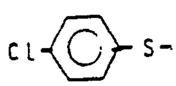 | $CH_3$ | $CH_3$ | 2,4-$Cl_2$ |
| 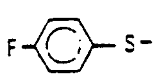 | $CH_3$ | $CH_3$ | 2,4-$Cl_2$ |
| 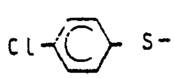 | $CH_3$ | $CH_3$ | 3,4-$Cl_2$ |
| 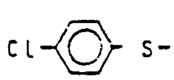 | $CH_3$ | $CH_3$ | 2-$CH_3$,4-Cl |
| 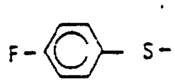 | $CH_3$ | $CH_3$ | 3,4-$Cl_2$ |

(Fortsetzung)

| $R^1$ | $R^2$ | $R^3$ | $Y_m$ |
|---|---|---|---|
| 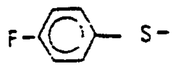 | $CH_3$ | $CH_3$ | $2-CH_3,4-Cl$ |
| 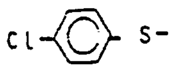 | $CH_3$ | $CH_3$ | 2-Cl |
| 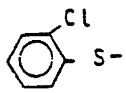 | $CH_3$ | $CH_3$ | 2-Cl |
| 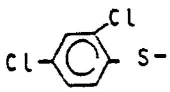 | $CH_3$ | $CH_3$ | 4-Cl |
| 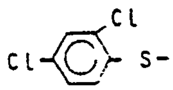 | $CH_3$ | $CH_3$ | $2,4-Cl_2$ |
| 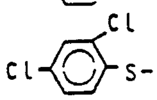 | $CH_3$ | $CH_3$ | 2-Cl |
| 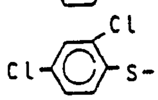 | $CH_3$ | $CH_3$ | 4-F |
| 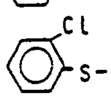 | $CH_3$ | $CH_3$ | 4-F |
| 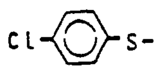 | $CH_3$ | $CH_3$ | 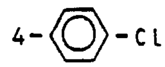 |
| 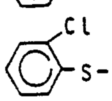 | $CH_3$ | $CH_3$ | 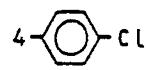 |
| 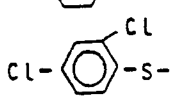 | $CH_3$ | $CH_3$ | 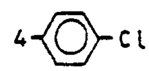 |
| 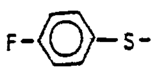 | $CH_3$ | $CH_3$ | 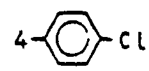 |
| 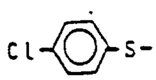 | $CH_3$ | $CH_3$ | 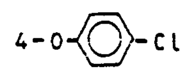 |
| 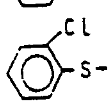 | $CH_3$ | $CH_3$ | 4-Cl |
| 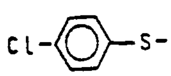 | $CH_3$ | $CH_3$ | 2-F |
| 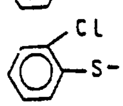 | $CH_3$ | $CH_3$ | 2-F |
| 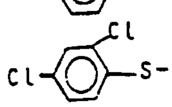 | $CH_3$ | $CH_3$ | 2-F |
| 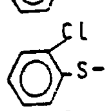 | $CH_3$ | $CH_3$ | $2,4-Cl_2$ |
| 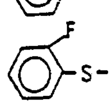 | $CH_3$ | $CH_3$ | $2,4-Cl_2$ |
| 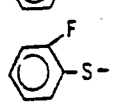 | $CH_3$ | $CH_3$ | 4-Cl |

(Fortsetzung)

| $R^1$ | $R^2$ | $R^3$ | $Y_m$ |
|---|---|---|---|
| 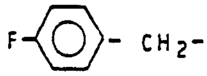 | $CH_3$ | $CH_3$ | 2-Cl |
| 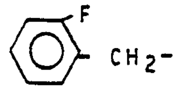 | $CH_3$ | $CH_3$ | 2-Cl |
| 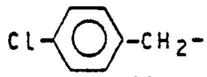 | $CH_3$ | $CH_3$ | 3-Cl |
| 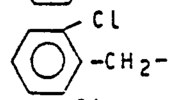 | $CH_3$ | $CH_3$ | 3,4-$Cl_2$ |
| 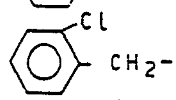 | $CH_3$ | $CH_3$ | 3-Cl |
| 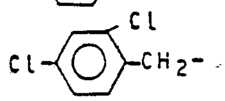 | $CH_3$ | $CH_3$ | 3-Cl |
| 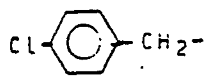 | $CH_3$ | $CH_3$ | 2,4-$Cl_2$ |
| 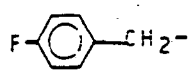 | $CH_3$ | $CH_3$ | 2,4-$Cl_2$ |
| 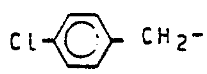 | $CH_3$ | $CH_3$ | 3,4-$Cl_2$ |
| 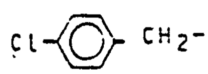 | $CH_3$ | $CH_3$ | 2-$CH_3$,4-Cl |
| 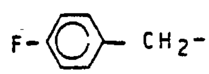 | $CH_3$ | $CH_3$ | 3,4-$Cl_2$ |
| 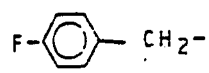 | $CH_3$ | $CH_3$ | 2-$CH_3$,4-Cl |
| 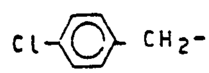 | $CH_3$ | $CH_3$ | 2-Cl |
| 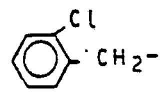 | $CH_3$ | $CH_3$ | 2-Cl |
| 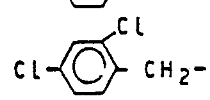 | $CH_3$ | $CH_3$ | 4-Cl |
| 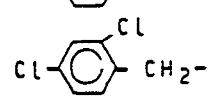 | $CH_3$ | $CH_3$ | 2,4-$Cl_2$ |
| 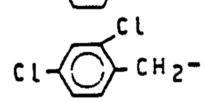 | $CH_3$ | $CH_3$ | 2-Cl |
| 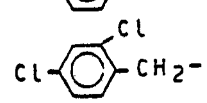 | $CH_3$ | $CH_3$ | 4-F |
| 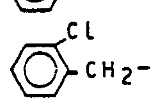 | $CH_3$ | $CH_3$ | 4-F |
| 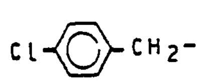 | $CH_3$ | $CH_3$ | 4-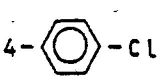 |

(Fortsetzung)

| R1 | R2 | R3 | Ym |
|---|---|---|---|
| 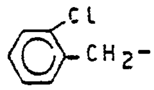 | $CH_3$ | $CH_3$ | 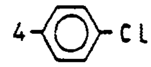 |
| 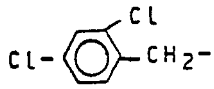 | $CH_3$ | $CH_3$ | 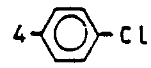 |
| 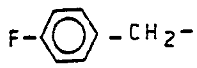 | $CH_3$ | $CH_3$ | 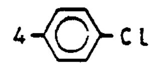 |
| 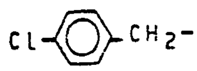 | $CH_3$ | $CH_3$ | 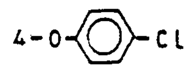 |
| 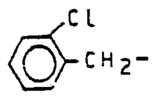 | $CH_3$ | $CH_3$ | 4-Cl |
| 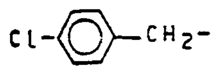 | $CH_3$ | $CH_3$ | 2-F |
| 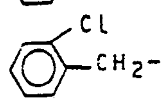 | $CH_3$ | $CH_3$ | 2-F |
| 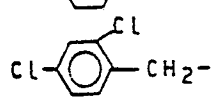 | $CH_3$ | $CH_3$ | 2-F |
| 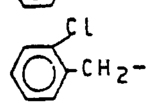 | $CH_3$ | $CH_3$ | 2,4-$Cl_2$ |
| 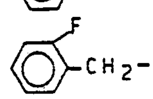 | $CH_3$ | $CH_3$ | 2,4-$Cl_2$ |
| 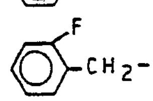 | $CH_3$ | $CH_3$ | 4-Cl |
| 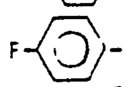 | $CH_3$ | $CH_3$ | 2-Cl |
| 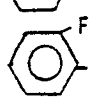 | $CH_3$ | $CH_3$ | 2-Cl |
| 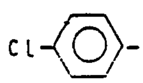 | $CH_3$ | $CH_3$ | 3-Cl |
| 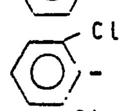 | $CH_3$ | $CH_3$ | 3,4-$Cl_2$ |
| 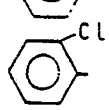 | $CH_3$ | $CH_3$ | 3-Cl |
| 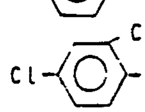 | $CH_3$ | $CH_3$ | 3-Cl |
| 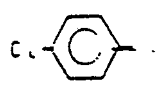 | $CH_3$ | $CH_3$ | 2,4-$Cl_2$ |
| 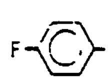 | $CH_3$ | $CH_3$ | 2,4-$Cl_2$ |
| 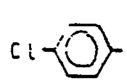 | $CH_3$ | $CH_3$ | 3,4-$Cl_2$ |

(Fortsetzung)

| $R^1$ | $R^2$ | $R^3$ | $Y_m$ |
|---|---|---|---|
| 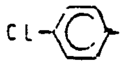 | $CH_3$ | $CH_3$ | 2-$CH_3$,4-Cl |
| 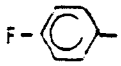 | $CH_3$ | $CH_3$ | 3,4-$Cl_2$ |
| 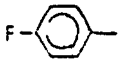 | $CH_3$ | $CH_3$ | 2-$CH_3$,4-Cl |
| 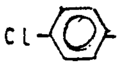 | $CH_3$ | $CH_3$ | 2-Cl |
| 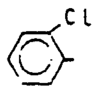 | $CH_3$ | $CH_3$ | 2-Cl |
| 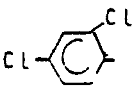 | $CH_3$ | $CH_3$ | 4-Cl |
| 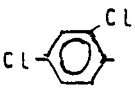 | $CH_3$ | $CH_3$ | 2,4-$Cl_2$ |
| 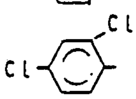 | $CH_3$ | $CH_3$ | 2-Cl |
| 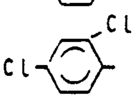 | $CH_3$ | $CH_3$ | 4-F |
| 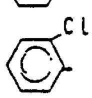 | $CH_3$ | $CH_3$ | 4-F |
| 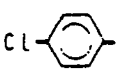 | $CH_3$ | $CH_3$ | 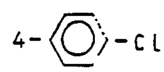 |
| 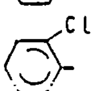 | $CH_3$ | $CH_3$ | 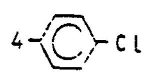 |
| 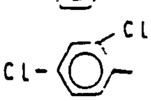 | $CH_3$ | $CH_3$ | 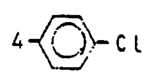 |
| 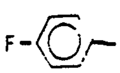 | $CH_3$ | $CH_3$ | 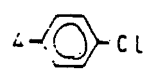 |
| 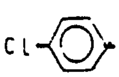 | $CH_3$ | $CH_3$ | 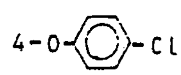 |
| 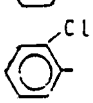 | $CH_3$ | $CH_3$ | 4-Cl |
| 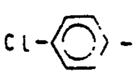 | $CH_3$ | $CH_3$ | 2-F |
| 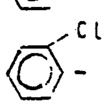 | $CH_3$ | $CH_3$ | 2-F |
| 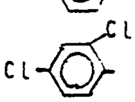 | $CH_3$ | $CH_3$ | 2-F |
| 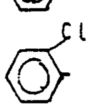 | $CH_3$ | $CH_3$ | 2,4-$Cl_2$ |

(Fortsetzung)

| $R^1$ | $R^2$ | $R^3$ | $Y_m$ |
|---|---|---|---|
| 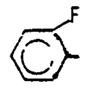 | $CH_3$ | $CH_3$ | 2,4-$Cl_2$ |
| 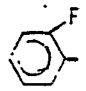 | $CH_3$ | $CH_3$ | 4-Cl |

Verwendet man beispielsweise 2-(4-Chlorphenyl)-2-(4-chlorphenyl-tert.-butyl)-oxiran und 1,2,4-Triazol als Ausgangsstoffe, so kann der Ablauf des erfindungsgemäßen Verfahrens durch das folgende Formelschema wiedergegeben werden :

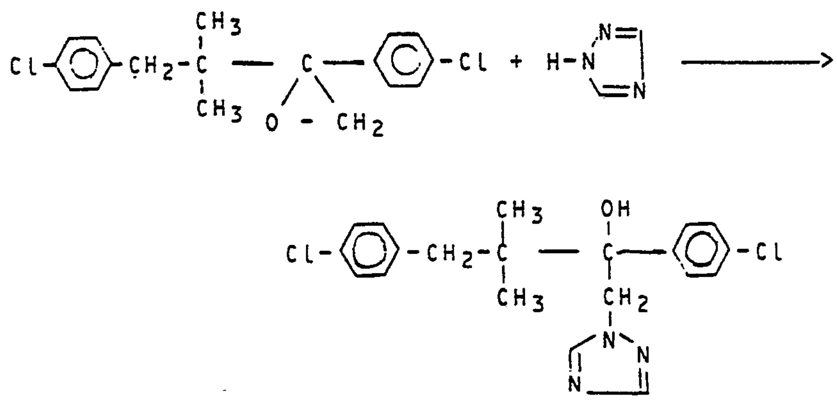

Verwendet man beispielsweise 2-(4-Chlorphenyl)-4-(4-chlorphenylthio)-3,3-dimethyl-1-(imidazol-1-yl)-2-butanol und Wasserstoffperoxid in Eisessig als Ausgangsstoffe, so kann der Reaktionsablauf der erfindungsgemäßen Oxidation durch das folgende Formelschema wiedergegeben werden :

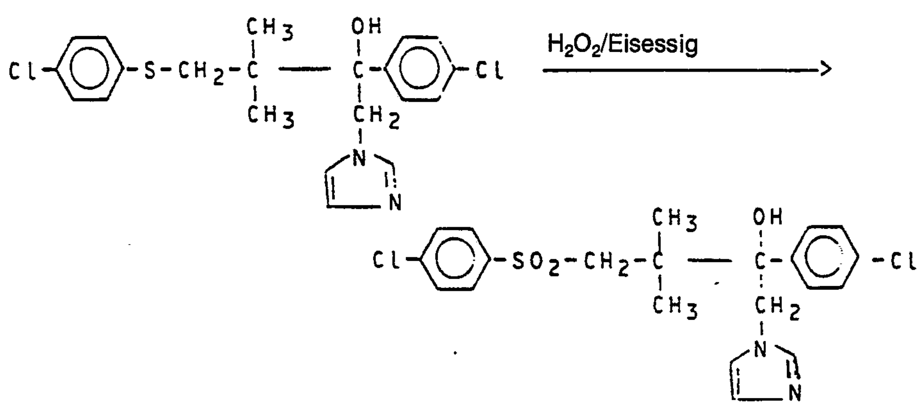


Die für die Durchführung des erfindungsgemäßen Verfahrens als Ausgangsstoffe zu verwendenden Oxirane sind durch die allgemeine Formel II allgemein definiert. In dieser Formel haben $R^1$, $R^2$, $R^3$, Y und der Index m vorzugsweise die Bedeutungen, die bereits im Zusammenhang mit der Beschreibung der erfindungsgemäßen Stoffe der allgemeinen Formel I vorzugsweise für diese Substituenten bzw. für den Index m genannt wurden.

Die Oxirane der allgemeinen Formel II sind noch nicht bekannt. Sie können jedoch in allgemein bekannter Art und Weise erhalten werden, indem man Ketone der allgemeinen Formel IV

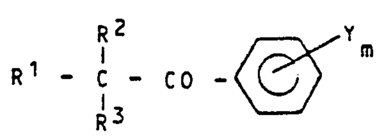

(IV)

in welcher $R^1$, $R^2$, $R^3$, Y und m die oben angegebene Bedeutung haben, entweder

α) mit Dimethyloxosulfonium-methylid der Formel V

$$\overset{\delta+\delta-}{(CH_3)_2SOCH_2} \quad (V)$$

in Gegenwart eines Verdünnungsmittels umsetzt, oder
β) mit Trimethylsulfonium-methylsulfat der Formel VI

$$[(CH_3)_3S^{\oplus}]\ CH_3SO_4^{\ominus} \quad (VI)$$

in Gegenwart eines inerten organischen Lösungsmittels sowie in Gegenwart einer Base umsetzt.

Die bei der Herstellung der Oxirane der allgemeinen Formel II als Ausgangsstoffe benötigten Ketone der allgemeinen Formel IV sind teilweise bekannt [vgl. z. B. J. Am. Chem. Soc. 1981, S. 4200 ff ; Zh. Org. Khim. (1973), 544-46 ; Bull. Soc. Chim. France 1972, *7*, 2756-59 und Zh. Org. Khim. *12*, (1976) 967-69] ; bzw. lassen sie sich nach im Prinzip bekannten Verfahren herstellen.

Das bei der Verfahrensvariante (α) benötigten Dimethyloxosulfoniummethylid der Formel V ist bekannt [vgl. J. Am. Chem. Soc. *87*, 1363-1364 (1965)]. Es wird bei der obigen Umsetzung in frisch zubereitetem Zustand verarbeitet, indem man es in situ durch Umsetzung von Trimethyloxosulfoniumiodid mit Natriumhydrid bzw. Natriumamid in Gegenwart eines Verdünnungsmittels erzeugt.

Das bei der Verfahrensvariante (β) benötigte Trimethylsulfonium-methylsulfat der Formel VI ist ebenfalls bekannt [vgl. Heterocycles *8*, 397 (1977)]. Es wird bei der obigen Umsetzung ebenfalls in frisch hergestelltem Zustand eingesetzt, indem man es durch Reaktion von Dimethylsulfid mit Dimethylsulfat in situ erzeugt.

Bei der Variante (α) des Verfahrens zur Herstellung der Oxirane der allgemeinen Formel II kommt als Verdünnungsmittel vorzugsweise Dimethylsulfoxid infrage.

Die Reaktionstemperaturen können bei der oben beschriebenen Verfahrensvariante (α) innerhalb eines größeren Bereiches variiert werden. Im allgemeinen arbeitet man bei Temperaturen zwischen 20 und 80 °C.

Die Durchführung des Verfahrens zur Herstellung der Oxirane der allgemeinen Formel II nach der Variante (α) und die Aufarbeitung des bei dieser Synthese anfallenden Reaktionsgemisches erfolgen nach üblichen Methoden [vgl. J. Am. Chem. Soc. *87*, 1363-1364 (1965)].

Bei der Variante (β) zur Herstellung der Oxirane der allgemeinen Formel II kommt als inertes organisches Lösungsmittel vorzugsweise Acetonitril in Betracht.

Als Basen können bei der Verfahrensvariante (β) starke anorganische oder organische Basen verwendet werden. Vorzugsweise kommt Natriummethylat infrage.

Die Reaktionstemperaturen können bei der oben beschriebenen Verfahrensvariante (β) innerhalb eines bestimmten Bereiches variiert werden. Im allgemeinen arbeitet man bei Temperaturen zwischen 0 und 60 °C, vorzugsweise bei Raumtemperatur.

Die Durchführung des Verfahrens zur Herstellung der Oxirane der allgemeinen Formel II nach der Variante (β) und die Aufarbeitung des bei dieser Synthese anfallenden Reaktionsproduktes erfolgen nach üblichen Methoden [vgl. Heterocycles *8*, 397 (1977)].

Die Oxirane der allgemeinen Formel II können bei dem erfindungsgemäßen Verfahren gegebenenfalls ohne Isolierung direkt weiter umgesetzt werden.

Die außerdem für das erfindungsgemäße Verfahren als Ausgangsstoffe zu verwendenden Azole sind durch die allgemeine Formel III allgemein definiert. In dieser Formel steht X vorzugsweise für die Bedeutungen, die bereits in der Erfindungsdefinition für diesen Substituenten genannt wurden. M steht vorzugsweise für Wasserstoff, Natrium oder Kalium.

Die für der Durchführung einer eventuellen erfindungsgemäßen Oxidation als Ausgangsstoffe zu verwendenten Verbindungen sind durch die allgemeine Formel Ia allgemein definiert. Die Verbindungen der allgemeinen Formel Ia sind erfindungsgemäße Stoffe.

Die erfindungsgemäße Oxidation erfolgt durch die Umsetzung mit üblichen anorganischen oder organischen Oxidationsmitteln. Hierzu gehören vorzugsweise organische Persäuren, wie z. B. Peressigsäure, p-Nitroperbenzoesäure, m-Chlorperbenzoesäure ; anorganische Persäuren, wie z. B. Periodsäure ; weiterhin Wasserstoffperoxid in Eisessig oder Methanol, Kaliumpermanganat und Chromsäure.

Als Verdünnungsmittel kommen für das erfindungsgemäße Verfahren unter den Reaktionsbedingungen inerte organische Lösungsmittel infrage. Hierzu gehören vorzugsweise Alkohole, wie z. B. Ethanol, Methoxyethanol oder Propanol ; Ketone, wie z. B. 2-Butanon ; Nitrile, wie z. B. Acetonitril ; Ester, wie z. B. Essigester ; Ether, wie z. B. Dioxan ; aromatische Kohlenwasserstoffe, wie z. B. Benzol und Toluol ; oder Amide, wie z. B. Dimethylformamid.

Als Basen kommen für die erfindungsgemäße Umsetzung alle üblicherweise verwendbaren anorganischen und organischen Basen in Betracht. Hierzu gehören vorzugsweise Alkalicarbonate, wie z. B. Natrium- und Kaliumcarbonat ; Alkalihydroxide, wie z. B. Natriumhydroxid ; Alkalialkoholate, wie z. B. Natrium- und Kalium-methylat und -ethylat ; Alkalihydride, wie z. B. Natriumhydrid ; sowie niedere tertiäre Alkylamine, Cycloalkylamine und Aralkylamine, wie insbesondere Triethylamin.

Die Reaktionstemperaturen können bei der Durchführung des erfindungsgemäßen Verfahrens in

einem größeren Bereich variiert werden. Im allgemeinen arbeitet man bei temperaturen zwischen 0 und 200 °C, vorzugsweise zwischen 60 und 150 °C.

Bei der Durchführung des erfindungsgemäßen Verfahrens setzt man vorzugsweise auf 1 Mol Oxiran der allgemeinen Formel II 1 bis 2 Mol Azol der allgemeinen Formel III und gegebenenfalls 1 bis 2 Mol Base ein ; die Isolierung der Endprodukte erfolgt in allgemein üblicher Weise.

Die Reaktionstemperaturen können bei der Durchführung der erfindungsgemäßen Oxidation in einem größeren Bereich variiert werden. Im allgemeinen arbeitet man zwischen etwa —50 bis 100 °C, vorzugsweise zwischen —30 und 80 °C.

Bei der Durchführung der erfindungsgemäßen Oxidation setzt man auf 1 Mol der erfindungsgemäßen Verbindungen der allgemeinen Formel Ia etwa 1 bis 5 Mol Oxidationsmittel ein. Bei der Anwendung von 1 Mol Oxidationsmittel, wie m-Chlorperbenzoesäure in Methylenchlorid oder Wasserstoffperoxid in Acetanhydrid bei Temperaturen zwischen —30 bis + 30 °C, entstehen vorzugsweise die erfindungsgemäßen Verbindungen der allgemeinen Formel I mit der —SO-Gruppierung. Bei Ueberschuß an Oxidationsmittel und höheren Temperaturen (10 bis 80 °C) entstehen vorzugsweise die erfindungsgemäßen Verbindungen der allgemeinen Formel I mit der —$SO_2$-Gruppierung. Die Isolierung der Oxidationsprodukte erfolgt in üblicher Weise.

Die Säureadditionssalze der Verbindungen der allgemeinen Formel I können in einfacher Weise nach üblichen Salzbildungsmethoden, z. B. durch Lösen einer Verbindung der Allgemeinen Formel I in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Die erfindungsgemäß verwendbaren Verbindungen der allgemeinen Formel I und ihre Säureadditions-Salze weisen antimikrobielle, insbesondere starke antimykotische Wirkungen auf. Sie besitzen ein sehr breites antimykotisches Wirkungsspektrum, insbesondere gegen Dermatophyten und Sproßpilze sowie bi-phasische Pilze, z. B. gegen Candida-Arten, wie Candida albicans, Epidermohyton-Arten, wie Epidermophyton floccosum, Aspergillus-Arten, wie Aspergillus niger und Aspergillus fumigatus, Trichophyton-Arten, wie Trichophyton mentagrophytes, Microsporon-Arten, wie Microsporon felineum sowie Torulopsis-Arten, wie Torulopsis glabrata. Die Aufzählung dieser Mikroorganismen stellt keinesfalls eine Beschränkung der bekämpfbaren Keime dar, sondern hat nur erläuternden Charakter.

Als Indikationsgebiete in der Humanmedizin können beispielsweise genannt werden :

Dermatomykosen und Systemmykosen durch Trichophyton mentagrophytes und andere Trichophytonarten, Mikrosporonarten, Epidermophyton floccosum, Sproßpilze und biphasische Pilze sowie Schimmelpilze hervorgerufen.

Als Indikationsgebiet in der Tiermedizin können beispielsweise aufgeführt werden :

Alle Dermatomykosen und Systemmykosen, insbesondere solche, die durch die obengenannten Erreger hervorgerufen werden.

Zur Erfindung gehören pharmazeutische Zubereitungen, die neben nicht toxischen, inerten pharmazeutisch geeigneten Trägerstoffen einen oder mehrere erfindungsgemäße Wirksstoffe enthalten oder die aus einem oder mehreren erfindungsgemäßen Wirkstoffen bestehen sowie Verfahren zur Herstellung dieser Zubereitungen.

Zur Erfindung gehören auch pharmazeutische Zubereitungen in Dosierungseinheiten. Dies bedeutet, daß die Zubereitungen in Form einzelner Teile, z. B. Tabletten, Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können z. B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbsfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z. B. Stärken, Milchzucker, Rohrzucker, Glukose, Mannit und Kieselsäure, (b) Bindemittel, z. B. Carboxymethylcellulose, Alginate, Gelatine, Polyvinylpyrrolidon, (c) Feuchthaltemittel, z. B. Glycerin, (d) Sprengmittel, z. B. Agar-Agar, Calciumcarbonat und Natriumbicarbonat, (e) Lösungsverzögerer, z. B. Paraffin und (f) Resorptionsbeschleuniger, z. B. quartenäre Ammoniumverbindungen, (g) Netzmittel, z. B. Cetylalkohol, Glycerinmonostearat, (h) Adsorptionsmittel, z. B. Kaolin und Bentonit und (i) Gleitmittel, z. B. Talkum, Calcium- und Magnesiumstearat und feste Polyäthylenglykole oder Gemische der unter (a) bis (i) aufgeführten Stoffe.

Den Tabletten, Dragees, Kapseln, Pillen und Granulate können mit den üblichen gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z. B. Polymersubstanzen und Wachse verwendet werden können.

Der oder die Wirkstoffe können gegebenenfalls mit einem oder mehreren der oben angegebenen Trägerstoffen auch in mikroverkapselter Form vorliegen.

Suppositorien können neben dem oder den Wirkstofen die üblichen wasserlöslichen oder wasserunlöslichen Trägerstoffe enthalten, z. B. Polyäthylenglykole, Fette, z. B. Kakaofett und höhere Ester (z. B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z. B. tierische und pflanzlische Fette, Wachse, Paraffine, Stärke, Tragant, Cellulosederivate, Polyäthylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z. B. Milchzucker, Talkum, Kieselsäure, Aluminiumhydroxid, Calciumsilikat und Polyamidpulver oder Gemische dieser Stoffe. Sprays können zusätzlich die üblichen Treibmittel z. B. Chlorfluorkohlenwasserstoffe enthalten.

Lösungen und Emulsionen können neben dem oder den Wirkstoffen die üblichen Trägerstoffe wie Lösungsmittel, Lösungsvermittler und Emulgatoren, z. B. Wasser, Äthylalkohol, Isopropylalkohol, Äthylcarbonat, Äthylacetat, Benzylalkohol, Benzylbenzoat, Propylenglykol, 1,3-Butylenglykol, Dimethylformamid, Oele, insbesondere Baumwollsaatöl, Erdnußöl, Meiskeimöl, Olivenöl, Ricinusöl und Sesamöl, Glycerin, Glycerinformal, Tetrahydrofurfurylalkohol, Polyäthylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z. B. Wasser, Äthylalkohol, Propylenglykol, Suspendiermittel, z. B. äthoxylierte Isostearylalkohole, Polyoxyäthylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmackverbessernde Zusätze, z. B. Pfefferminzöl und Eukalyptusöl und Süßmittel, z. B. Saccharin enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z. B. durch Mischen des oder der Wirkstoffe mit dem oder den Trägerstoffen.

Die Wirkstoffe oder die pharmazeutischen Zubereitungen können lokal, oral, parenteral, intraperitoneal und/oder rectal, vorzugsweise parenteral, insbesondere intravenös appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 10 bis etwa 300, vorzugsweise 50 bis 200 mg/kg Körpergewicht je 24 Stunden, gegebenenfalls in Form mehrerer Einzelgaben zur Erzielung der gewünschten Ergebnise zu verabreichen.

Es kann jedoch erforderlich sein, von den genannten Dosierungen abzuweichen und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart des Wirkstoffe kann durch jeden Fachmann aufgrund seines Fachwissens leicht erfolgen.

Herstellungsbeispiele

Beispiel 1

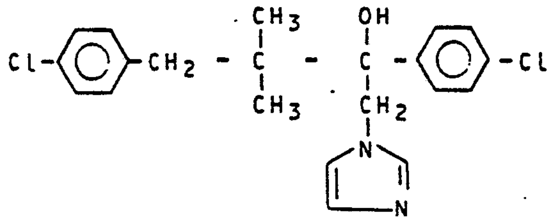

Zu einer Lösung von 7,7 g (0,108 Mol) Imidazol-Natrium in 60 ml n-Propanol werden bei Rückflußtemperatur 30 g (0,093 5 Mol) 2-(4-Chlorphenyl)-2-(4-chlorphenyl-tert.-butyl)-oxiran in 40 ml n-Propanol getropft. Man läßt das Reaktionsgemisch 48 Stunden unter Rückfluß nachrühren, kühlt ab, versetzt mit

Wasser und extrahiert mit Methylenchlorid. Die organische Phase wird über Natriumsulfat getrocknet und im Vakuum eingeengt. Der ölige Rückstand wird in Diisopropylether verrührt. Der entstehende kristalline Niederschlag wird abgesaugt und getrocknet. Man erhält 12,7 g (35 % der Theorie) 2,4-Bis(4-chlorphenyl)-3,3-dimethyl-1-(imidazol-1-yl)-2-butanol vom Schmelzpunkt 174 °C.

Herstellung des Ausgangsproduktes

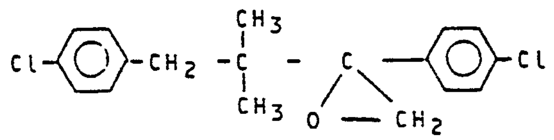

Eine Lösung von 59,2 g (0,47 Mol) Dimethylsulfat und 32 g (0,517 Mol) Dimethylsulfid in 270 ml Acetonitril läßt man 5 Tage bei Raumtemperatur rühren. Innerhalb von ca. 2 Stunden läßt man dann bei 20 bis 25 °C eine Lösung von 81,5 g (0,265 5 Mol) 4-Chlorphenyl-(4-chlorphenyl-tert.-butyl-keton in 80 ml Acetonitril zutropfen. Bei gleicher Temperatur werden 28,7 g (0,53 Mol) Natriummethylat zugegeben. Man läßt das gesamte Reaktionsgemisch 12 Stunden nachrühren und engt danach im Vakuum ein. Der Rückstand wird mit einem Gemisch aus 200 ml Essigester und 150 ml Wasser über Nacht verrührt. Die organische Phase wird abgetrennt, über Natriumsulfat getrocknet und im Vakuum eingeengt. Man erhält 72,6 g (85,2 % der Theorie) rohes 2-(4-Chlorphenyl)-2-(4-chlorphenyl-tert.-butyl)-oxiran, das direkt weiter umgesetzt wird.

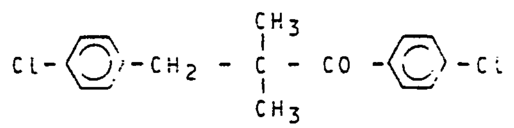

85 g (0,466 Mol) 4-Chlorphenyl-isopropyl-keton, 31,3 g (0,56 Mol) Kaliumhydroxid und 5 g Tetrabutylammoniumbromid in 120 ml Toluol werden auf Rückflußtemperatur erhitzt und tropfenweise mit einer Lösung von 75 g (0,466 Mol) 4-Chlorbenzylchlorid in 60 ml Toluol versetzt. Man läßt das Reaktionsgemisch 12 Stunden unter Rückfluß nachrühren, kühlt ab, wäscht mit Wasser, trocknet die organische Phase über Natriumsulfat und engt im Vakuum ein. Man erhält 81,5 g (60 % der Theorie) 4-Chlorphenyl-(4-chlorphenyl-tert.-butyl)-keton vom Brechungsindex $n_D^{20}$ = 1,571 1.

Beispiel 2

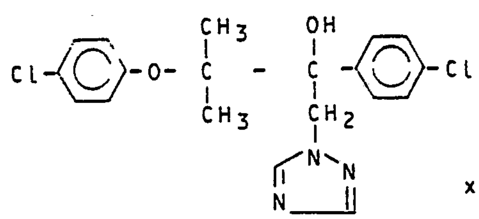

x HCl

Eine Lösung von 30 g (0,093 Mol) 2-(4-Chlorphenyl)-2-[2-(p-chlorphenoxy)-prop-2-yl]-oxiran in 40 ml n-Propanol wird bei Raumtemperatur zu einer Lösung von 7,6 g (0,107 Mol) 1,2,4-Triazol-Natrium in 60 ml n-Propanol getropft. Man läßt das Reaktionsgemisch 48 Stunden bei Rückflußtemperatur nachrühren, kühlt ab, versetzt mit Wasser und extrahiert mit Methylenchlorid. Die organische Phase wird über Natriumsulfat getrocknet und im Vakuum eingeengt. Der ölige Rückstand wird säulenchromatographisch gereinigt. Man erhält 6,7 g (18,4 % der Theorie) 3-(4-Chlorphenoxy)-2-(4-chlorphenyl)-3-methyl-1-(1,2,4-triazol-1-yl)-2-butanol. Diese werden bei Raumtemperatur mit 20 ml gesättigter Chlorwasserstoff/Ether-Lösung verrührt. Der ausfallende Niederschlag wird abgesaugt, mit wenig Ether nachgewaschen und im Vakuum bei 40 °C getrocknet. Man erhält 6,5 g (89 % der Theorie bezogen auf eingesetzte Base) 3-(4-Chlorphenoxy)-2-(4-chlorphenyl)-3-methyl-1-(1,2,4-triazol-1-yl)-2-butanolhydrochlorid vom Schmelzpunkt 135 °C.

Herstellung des Ausgangsproduktes

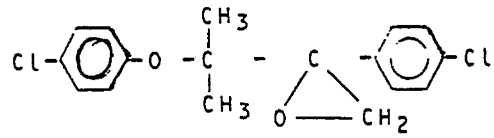

Eine Lösung von 59,2 g (0,47 Mol) Dimethylsulfat und 32 g (0,517 Mol) Dimethylsulfid in 270 ml Acetonitril läßt man 5 Tage bei Raumtemperatur rühren. Innerhalb von ca.

2 Stunden läßt man dann bei 20 bis 25 °C eine Lösung von 87 g 4-Chlorphenyl-[2-(p-chlorphenoxy)-prop-2-yl]-keton in 80 ml Acetonitril zutropfen. Bei gleicher Temperatur trägt man 28,7 g (0,53 Mol) Natriummethylat ein, läßt 12 Stunden nachrühren und engt anschließend ein. Der Rückstand wird mit einem Gemisch aus 200 ml Essigester und 150 ml Wasser über Nacht verrührt. Die organische Phase wird abgetrennt, über Natriumsulfat getrocknet und im Vakuum eingeengt. Man erhält 49 g (76 % der Theorie) rohes 2-(4-Chlorphenyl)-2-[2-(p-chlorphenoxy)-prop-2-yl]-oxiran, das direkt weiter umgesetzt wird.

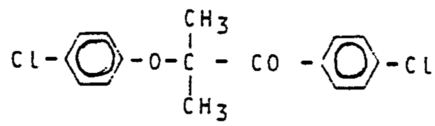

52 g (0,398 2 Mol) p-Chlorphenol und 55 g (0,398 2 Mol) Kaliumcarbonat in 400 ml Toluol werden 2 Stunden unter Rückfluß am Wasserabscheider erhitzt. Man kühlt auf 40 °C ab und versetzt tropfenweise mit einer Lösung von 2-Brom-prop-2-yl-(4-chlorphenyl)-keton in 170 ml Toluol. Dieses Reaktionsgemisch läßt man 5 Stunden bei100 °C nachrühren, kühlt anschließend ab, versetzt mit Wasser und trennt die organische Phase ab. Diese wird mit verdünnter Natronlauge sowie Wasser gewaschen, über Natriumsulfat getrocknet und eingeengt. Man erhält 87 g (85 % der Theorie) rohes 4-Chlorphenyl-[2-(p-chlorphenoxy)-prop-2-yl]-keton, das direkt weiter umgesetzt wird.

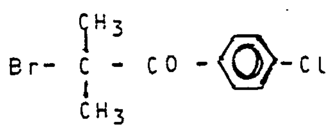

Zu 65,5 g (0,36 Mol) 4-Chlorphenyl-isopropyl-keton in 200 ml Chloroform gibt man 1 ml Bromwasserstoff/Eisessig zu und läßt dann bei 30 °C 57,5 g (0,36 Mol) Brom zutropfen. Man läßt 30 Minuten bei Raumtemperatur nachrühren und engt danach im Vakuum ein. Man erhält 86,6 g (92 % der Theorie) rohes 2-Brom-prop-2-yl-(4-chlorphenyl)-keton, das direkt weiter umgesetzt wird.

In entsprechender Weiser und gemäß dem erfindungsgemäßen Verfahren werden die folgenden Verbindungen der allgemeinen Formel I

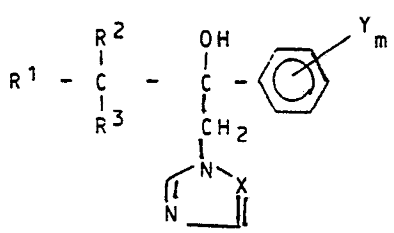

(I)

erhalten :

| Bsp. Nr. | $R^1$ | $R^2$ | $R^3$ | X | $Y_m$ | Schmelzpunkt (°C) |
|---|---|---|---|---|---|---|
| 3 | 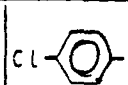 | $CH_3$ | $CH_3$ | CH | 4-Cl | 138 |
| 4 | 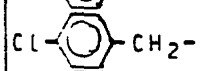 | $CH_3$ | $CH_3$ | N | 4-Cl | 137 |
| 5 | 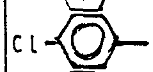 | $CH_3$ | $CH_3$ | N | 4-Cl | 109 |
| 6 | 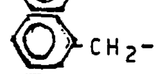 | $CH_3$ | $CH_3$ | N | 4-F | 120 |
| 7 | 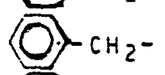 | $CH_3$ | $CH_3$ | CH | 4-F | 118-120 |
| 8 | 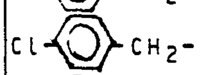 | $CH_3$ | $CH_3$ | CH | 4-F | 165-167 |
| 9 | 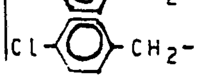 | $CH_3$ | $CH_3$ | N | 4-F | 124 |

(Fortsetzung)

| Bsp. Nr. | $R^1$ | $R^2$ | $R^3$ | X | $Y_m$ | Fp (°C) |
|---|---|---|---|---|---|---|
| 10 | 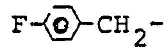 | $CH_3$ | $CH_3$ | CH | 4-Cl | 126 |
| 11 | 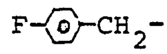 | $CH_3$ | $CH_3$ | N | 4-Cl | 112 |
| 12 | 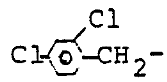 | $CH_3$ | $CH_3$ | CH | 4-F | 93 |
| 13 | 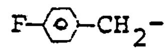 | $CH_3$ | $CH_3$ | CH | - | 164-65 |
| 14 | 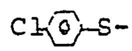 | $CH_3$ | $CH_3$ | N | 4-Cl | 74-76 |
| 15 | 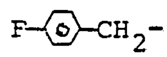 | $CH_3$ | $CH_3$ | N | - | 102-04 |
| 16 | 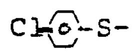 | $CH_3$ | $CH_3$ | CH | 4-Cl | 94 |
| 17 | 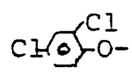 | $CH_3$ | $CH_3$ | CH | 4-Cl | 80-82 |
| 18 | 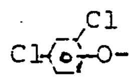 | $CH_3$ | $CH_3$ | N | 4-Cl | 96-98 |
| 19 | 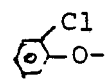 | $CH_3$ | $CH_3$ | N | 4-Cl | 114-16 |
| 20 | 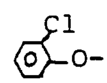 | $CH_3$ | $CH_3$ | CH | 4-Cl | 194 |
| 21 | 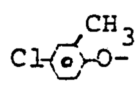 | $CH_3$ | $CH_3$ | N | 4-Cl | 117 |
| 22 | 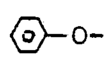 | $CH_3$ | $CH_3$ | N | 4-Cl | 82 |
| 23 | 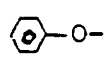 | $CH_3$ | $CH_3$ | CH | 4-Cl | 188 |
| 24 | 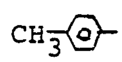 | $CH_3$ | $CH_3$ | N | 4-Cl | 62 |
| 25 | 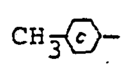 | $CH_3$ | $CH_3$ | CH | 4-Cl | 106 |
| 26 | 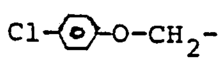 | $C_2H_5$ | $CH_3$ | N | 4-Cl | 104-06 |
| 27 | 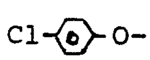 | $C_2H_5$ | $CH_3$ | N | 4-Cl | 182-84 (x HCl) |
| 28 | 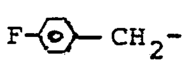 | $CH_3$ | $CH_3$ | N | 4-F | 118 |
| 29 | 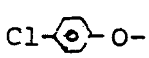 | $CH_3$ | $CH_3$ | N | 4-F | 82 |

(Fortsetzung)

| Bsp. Nr. | $R^1$ | $R^2$ | $R^3$ | X | $Y_m$ | Fp (°C) |
|---|---|---|---|---|---|---|
| 30 | 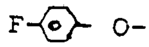 | $CH_3$ | $CH_3$ | N | 4-F | 78 |
| 31 | 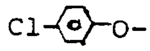 | $CH_3$ | $CH_3$ | CH | 4-F | 154 |
| 32 | 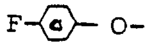 | $CH_3$ | $CH_3$ | CH | 4-F | 178 |
| 33 | 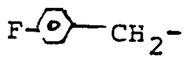 | $CH_3$ | $CH_3$ | CH | 4-F | 50 |
| 34 | 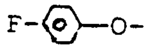 | $C_2H_5$ | $CH_3$ | N | 4-F | 114 |
| 35 | 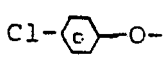 | $C_2H_5$ | $CH_3$ | N | 4-F | 66 |

Verwendungsbeispiele

In den nachfolgenden Beispielen werden die nachstehend angegebenen Verbindungen als Vergleichssubstanzen eingesetzt :

(A)

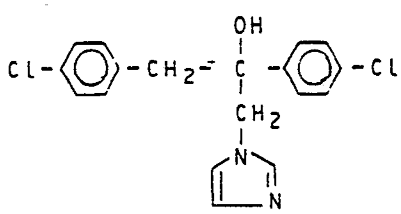

(B)

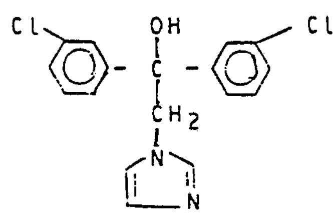

(C)

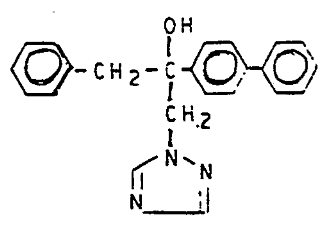

Beispiel A

Antimykotische in-vitro-Wirksamkeit

Versuchsbeschreibung :

Die in-vitro-Prüfungen wurden im Reihenverdünnungstest mit Keiminokula von durchschnittlich 5 × $10^4$ Keimen/ml Substrat durchgeführt. Als Nährmedium dienten

a) für Dermatophyten und Schimmelpilze : Sabouraud's milieu d'épreuve
b) für Hefen : Fleischextrakt-Traubenzucker-Bouillon.

Die Bebrütungstemperatur betrug 20 °C, die Bebrütungsdauer lag bei 24 bis 96 Stunden bei Hefen und 96 Stunden bei Dermatophyten und Schimmelpilzen.

In diesem Test zeigen insbesondere die Verbindungen der Beispiele 1, 2, 3, 4 und 5 eine bessere antimykotische Wirkung als die aus dem Stand der Technik bekannten Verbindungen (A), (B) und (C).

Tabelle A

Antimykotische in-vitro-Wirksamkeit

| Wirkstoff | MHK-Werte in γ/ml Nährmedium bei | | | |
|---|---|---|---|---|
| | Trichophyton mentagr. | Microsporum canis | Candida albicans | Torulopsis glabrata |
| (A) (bekannt) | 32 | 64 | 16 | 32 |
| (B) (bekannt) | 16 | 32 | 16 | 16 |
| (C) (bekannt) | <1 | 16 | >64 | - |
| Verbindungen gemäß Herst.Bsp. | | | | |
| 1 | ≤1 | 8 | 4 | ≤1 |
| 2 | ≤1 | 16 | 16 | 8 |
| 3 | 4 | 16 | 4 | 16 |
| 4 | ≤1 | 16 | ≤1 | 4 |
| 5 | ≤1 | ≤1 | 16 | 4 |

Beispiel B

Antimykotische in-vivo-Wirksamkeit (oral) bei Mäusecandidose

Versuchsbeschreibung :

Mäuse vom Typ SPF-$CF_1$ wurden intravenös mit 1-2 × $10^6$ logarithmisch wachsenden Candida-Zellen, die in physiologischer Kochsalzlösung suspendiert werden, infiziert. Eine Stunde vor und sieben Stunden nach der Infektion werden die Tiere mit jeweils 25-100 mg/kg Körpergewicht der Präparate oral behandelt.

Ergebnis :

Unbehandelte Tiere starben 3 bis 6 Tage post infektionem. Die Ueberlebensrate am 6. Tag post infektionem betrug bei unbehandelten Kontrolltieren etwa 5 %.

In diesem Test zeigten z. B. die erfindungsgemäßen Verbindungen 1 und 4.

Eine bessere Wirkung als die aus dem Stand der Technik bekannten Verbindungen (A), (B) und (C).

Zeichenerklärung :

+++++ = sehr gute Wirkung = 90 % Ueberlebende am 6. Tag p. i.
++++ = gute Wirkung = 80 % Ueberlebende am 6. Tag p. i.
+++ = Wirkung = 60 % Ueberlebende am 6. Tag p. i.
++ = schwache Wirkung = 40 % Ueberlebende am 6. Tag p. i.
+ = Spur Wirkung =
k. W. = keine Wirkung

Tabelle B

Antimykotische in-vivo-Wirksamkeit (oral) bei Mäusecandidose

| Wirkstoff | Wirkung |
|---|---|
| (A) (bekannt) | + |
| (B) (bekannt) | k.W. |
| (C) (bekannt) | k.W. |
| Verbindungen gemäß Herst.Bsp. | |
| 1 | +++ |
| 4 | +++++ |

Beispiel C/Formulierungen

1) Lösung :

| | |
|---|---|
| Wirkstoff gemäß allgemeiner Formel I : | 10 g |
| Alkohol, rein (96 %-ig) : | 300 g |
| Isopropylmyristat : | 526 g |
| | 836 g |

2) Creme :

| | |
|---|---|
| Wirkstoff gemäß allgemeiner Formel I : | 10 g |
| Arlacel 60 : | 20 g |
| (Sorbitan-monostearat) Tween 60 : | 15 g |
| (Polyoxyethylen (20)-sorbitanmonostearat) | |
| Walrat, künstlich : | 30 g |
| (Mischung von Estern von gesättigten Fettsäuren $C_{14}$-$C_{18}$ und Fettalkoholen $C_{14}$-$C_{18}$) | |
| Lanette O : | 100 g |
| (Gemisch aus Cetyl-Alkohol und Stearyl-Alkohol) | |
| Entanol G : | 135 g |
| (2-Octyl-dodecanol) | |
| Benzylalkohol : | 10 g |
| Wasser, entmineralisiert : | 680 g |
| | 1 000 g |

**Patentansprüche**

1. Substituierte Hydroxyalkyl-azole der allgemeinen Formel I

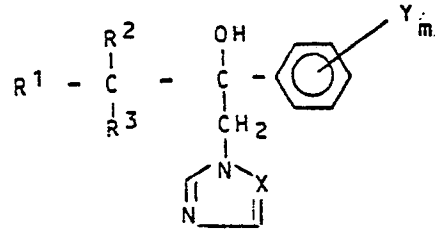

(I)

in welcher

$R^1$ für jeweils einfach bis zweifach, gleich oder verschieden substituiertes Phenyl, —O-Phenyl, —S-

Phenyl, —SO-Phenyl, —$SO_2$-Phenyl, —$CH_2$-Phenyl, —$CH_2$—O-Phenyl, —$CH_2$—S-Phenyl, —$CH_2$—SO-Phenyl oder —$CH_2$—$SO_2$-Phenyl, wobei als Phenylsubstituenten die Bedeutungen von Y genannt seien ;

$R^2$ für gradkettiges oder verzweigtes Alkyl mit 1 bis 4 Kohlenstoffatomen ;

$R^3$ für gradkettiges oder verzweigtes Alkyl mit 1 bis 4 Kohlenstoffatomen ;

X für ein Stickstoffatom oder die CH-Gruppe ;

Y für Halogen, Alkyl mit 1 bis 4 Kohlenstoffatomen, Cycloalkyl mit 5 bis 7 Kohlenstoffatomen, Alkoxy und Alkylthio mit jeweils 1 bis 4 Kohlenstoffatomen, Halogenalkyl und Halogenalkoxy sowie Halogenalkylthio mit jeweils 1 bis 2 Kohlenstoff- und 1 bis 5 gleichen oder verschiedenen Halogenatomen, sowie für jeweils gegebenenfalls durch Halogen und Alkyl mit 1 bis 2 Kohlenstoffatomen substituiertes Phenyl, Phenoxy, Phenylalkyl sowie Phenylalkoxy mit 1 bis 2 Kohlenstoffatomen im Alkylteil bzw. im Alkoxyteil ; und

m für die Zahlen 0, 1, 2 oder 3

stehen.

2. Substituierte Hydroxyalkyl-azole der allgemeinen Formel I in Anspruch 1, in welcher

$R^1$ für jeweils einfach bis zweifach, gleich oder, verschieden substituiertes Phenyl, —O-Phenyl, —S-Phenyl, —SO-Phenyl, —$SO_2$-Phenyl, —$CH_2$-Phenyl, —$CH_2$—O-Phenyl, —$CH_2$—O-Phenyl, —$CH_2$—S-Phenyl, — —$CH_2$—SO-Phenyl oder —$CH_2$—$SO_2$-Phenyl steht, wobei als Phenylsubstituenten die Bedeutungen von Y genannt seien ;

$R^2$ für Methyl oder Ethyl steht,

$R^3$ für Methyl oder Ethyl steht ;

X für ein Stickstoffatom oder die CH-Gruppe steht ;

Y für Fluor, Chlor, Brom, Methyl, Isopropyl, tert.-Butyl, Cyclohexyl, Methoxy, Methylthio, Trifluormethyl, Trifluormethoxy, Trifluormethylthio sowie für gegebenenfalls durch Fluor, Chlor, Methyl substituiertes Phenyl, Phenoxy, Benzyl oder Benzyloxy steht ; und

m für die Zahlen 0, 1, 2 oder 3 steht.

3. Oxirane der allgemeinen Formel II

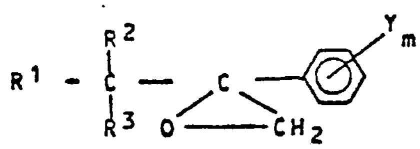

(II)

in welcher $R^1$, $R^2$, $R^3$, Y und m die oben angegebene Bedeutung haben.

4. 2,4-Bis(4-chlorphenyl)-3,3-dimethyl-1-(imidazol-1-yl)-2-butanol.

5. 3-(4-Chlorphenoxy)-2-(4-chlorphenyl)-3-methyl-1-(1,2,4-triazol-1-yl)-2-butanol.

6. 2-(4-Chlorphenyl)-2-(4-chlorphenyl-tert.butyl)-oxiran.

7. 2-(4-Chlorphenyl)-2-[2-(p-chlorphenoxy)-prop-2-yl] oxiran.

8. Verfahren zur Herstellung von substituierten Hydroxyalkyl-azolen der allgemeinen Formel I in Anspruch 1, dadurch gekennzeichnet, daß man Oxirane der allgemeinen Formel II

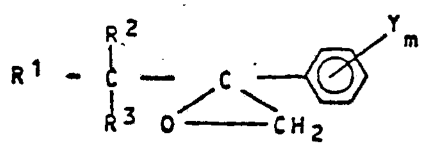

(II)

in welcher $R^1$, $R^2$, $R^3$, Y und m die oben angegebene Bedeutung haben, mit Azolen der allgemeinen Formel III

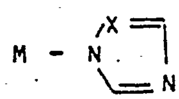

(III)

in welcher

X die oben angegebene Bedeutung hat

und

m für Wasserstoff oder ein Alkalimetall steht,

in Gegenwart eines Verdünnungsmittels und gegebenenfalls in Gegenwart einer Base umsetzt, und gegebenenfalls die so erhaltenen Hydroxyalkyl-azole der allgemeinen Formel Ia

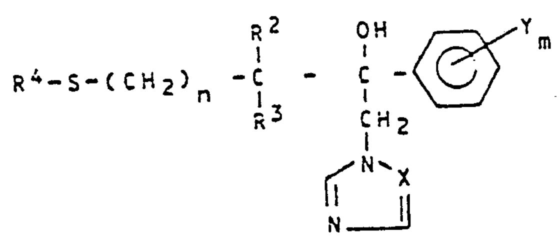

(Ia)

in welcher
$R^2$, $R^3$, X, Y und m die oben angegebene Bedeutung haben,
$R^4$ für gegebenenfalls durch Y substituiertes Phenyl steht und
n für die Zahlen 0 oder 1 steht,
nach bekannten Methoden in üblicher Weise oxidiert.

9. Arzneimittel, gekennzeichnet durch einen Gehalt an einem substituierten Hydroxyalkyl-azol der allgemeinen Formel I in Anspruch 1.

**Claims**

1. Substituted hydroxyalkyl-azoles of the general formula I

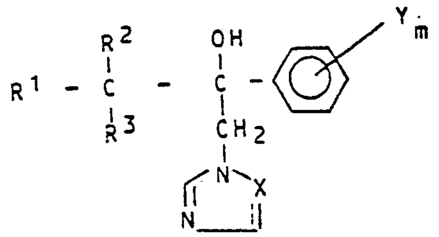

(I)

in which $R^1$ represents phenyl, —O-phenyl, —S-phenyl, —SO-phenyl, —$SO_2$-phenyl, —$CH_2$-phenyl, —$CH_2$—O-phenyl, —$CH_2$—S-phenyl, —$CH_2$—SO-phenyl or —$CH_2$—$SO_2$-phenyl, each of which is mono- to disubstituted by identical or different substituents, the meanings of Y being mentioned as the phenyl substituents ;

$R^2$ represents straight-chain or branched alkyl with 1 to 4 carbon atoms ;

$R^3$ represents straight-chain or branched alkyl with 1 to 4 carbon atoms ;

X represents a nitrogen atom or the CH group ;

Y represents halogen, alkyl with 1 to 4 carbon atoms, cycloalkyl with 5 to 7 carbon atoms, alkoxy and alkylthio each with 1 to 4 carbon atoms, halogenoalkyl and halogenoalkoxy and halogenoalkylthio each with 1 to 2 carbon atoms and 1 to 5 identical or different halogen atoms, and phenyl, phenoxy, phenylalkyl and phenylalkoxy with 1 to 2 carbon atoms in the alkyl part or in the alkoxy part, each of which is optionally substituted by halogen and alkyl with 1 to 2 carbon atoms, and

m represents the numbers 0, 1, 2 or 3.

2. Substituted hydroxyalkyl-azoles of the general formula I in claim 1, in which

$R^1$ represents phenyl, —O-phenyl, —S-phenyl, —SO-phenyl, —$SO_2$-phenyl, —$CH_2$-phenyl, —$CH_2$—O-phenyl, —$CH_2$—O-phenyl, —$CH_2$—S-phenyl, —$CH_2$—SO-phenyl or —$CH_2$—$SO_2$-phenyl, each of which is mono- to disubstituted by identical or different substituents, the meanings of Y being mentioned as the phenyl substituents ;

$R^2$ represents methyl or ethyl,

$R^3$ represents methyl or ethyl ;

X represents a nitrogen atom or the CH group ;

Y represents fluorine, chlorine, bromine, methyl, isopropyl, tert.-butyl, cyclohexyl, methoxy, methylthio, trifluoromethyl, trifluoromethoxy, trifluoromethylthio, and optionally fluorine-, chlorine-, or methyl-substituted phenyl, phenoxy, benzyl or benzyloxy ; and

m represents the numbers 0, 1, 2 or 3.

3. Oxiranes of the general formula II

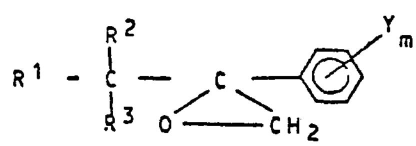

(II)

in which $R^1$, $R^2$, $R^3$, Y and m have the abovementioned meaning.

4. 2,4-Bis(4-chlorophenyl)-3,3-dimethyl-1-(imidazol-1-yl)-butan-2-ol.

5. 3-(4-Chlorophenoxy)-2-(4-chlorophenyl)-3-methyl-1-(1,2,4-triazol-1-yl)-butan-2-ol.

6. 2-(4-Chlorophenyl)-2-(4-chlorophenyl-tert.-butyl)-oxirane.

7. 2-(4-Chlorophenyl)-2-[2-(p-chlorophenoxy)-prop-2-yl]-oxirane.

8. Process for the preparation of substituted hydroxyalkyl-azoles of the general formula I in claim 1, characterised in that oxiranes of the general formula II

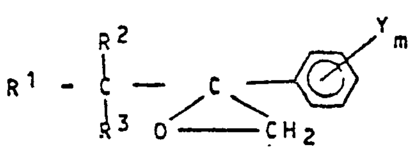

(II)

in which $R^1$, $R^2$, $R^3$, Y and m have the abovementioned meaning, are reacted with azoles of the general formula III

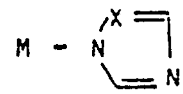

(III)

in which

X has the abovementioned meaning and

m represents hydrogen or an alkali metal,

in the presence of a diluent and, if appropriate, in the presence of a base, and, if appropriate, the hydroxyalkyl-azoles thus obtained, of the general formula Ia

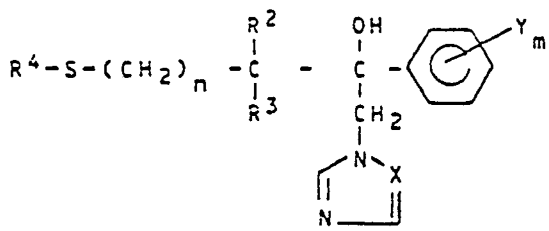

(Ia)

in which

$R^2$, $R^3$, X, Y and m have the abovementioned meaning,

$R^4$ represents optionally Y-substituted phenyl and

n represents the numbers 0 or 1,

are oxidised in a customary manner, by known methods.

9. Medicaments, characterised in that they contain a substituted hydroxyalkyl-azole of the general formula I in claim 1.

**Revendications**

1. Hydroxy-azoles substitués de formule générale I :

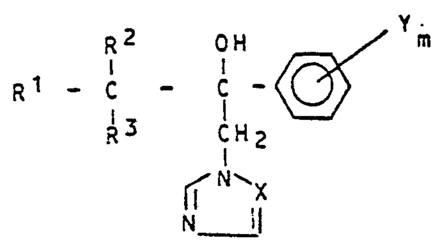

(I)

dans laquelle

$R^1$ représente un groupe phényle, —O-phényle, —O-phényle, —SO-phényle, —$SO_2$-phényle, —$CH_2$-phényle, —$CH_2$—O-phényle, —$CH_2$—S-phényle, —$CH_2$—SO-phényle ou —$CH_2$—$SO_2$-phényle, chaque fois substitué une à deux fois, de manière identique ou différente, et l'on peut indiquer comme substituants du groupe phényle les sens de Y ;

$R^2$ représente un groupe alkyle linéaire ou ramifié ayant 1 à 4 atomes de carbone ;

$R^3$ représente un groupe alkyle linéaire ou ramifié ayant 1 à 4 atomes de carbone ;

X représente un atome d'azote ou le groupe CH ;

Y représente un atome d'halogène, un groupe alkyle ayant 1 à 4 atomes de carbone, cycloalkyle ayant 5 à 7 atomes de carbone, alcoxy et alkylthio ayant à chaque fois 1 à 4 atomes de carbone, halogénoalkyle et halogénoalcoxy, ainsi que halogénoalkylthio ayant à chaque fois 1 à 2 atomes de carbone et 1 à 5 atomes d'halogènes identiques ou différents, ainsi qu'un groupe phényle, phénoxy, phénylalkyle et phénylalcoxy, ayant 1 à 2 atomes de carbone dans la partie alkyle ou dans la partie alcoxy et éventuellement substitué par de l'halogène et par un groupe alkyle ayant 1 à 2 atomes de carbone ; et

m représente les nombres 0, 1, 2 ou 3.

2. Hydroxy-azoles substitués de formule générale I de la revendication 1, dans laquelle :

$R^1$ représente un groupe phényle, —O-phényle, —S-phényle, —SO-phényle, —$SO_2$-phényle, —$CH_2$-phényle, —$CH_2$—O-phényle, —$CH_2$—S-phényle, —$CH_2$—SO-phényle ou —$CH_2$—$SO_2$-phényle, chaque fois substitué une à deux fois, de manière identique ou différente, et l'on peut indiquer comme substituants du groupe phényle les sens de Y ;

$R^2$ représente un groupe méthyle ou éthyle ;

$R^3$ représente un groupe méthyle ou éthyle ;

X représente un atome d'azote ou le groupe CH ;

Y représente un atome de fluor, de chlore ou de brome ou un groupe méthyle, isopropyle, tert-butyle, cyclohexyle, méthoxy, méthylthio, trifluorométhyle, trifluorométhoxy, trifluorométhylthio, ainsi qu'un groupe phényle, éventuellement substitué par du fluor, du chlore ou un groupe méthyle, phénoxy, benzyle ou benzyloxy ; et

m représente les nombres 0, 1 ou 3.

3. Oxirannes de formule générale II :

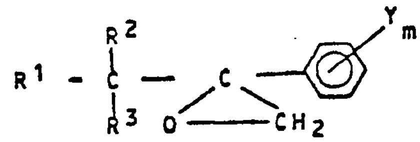

(II)

dans laquelle $R^1$, $R^2$, $R^3$, Y et m ont le sens indiqué ci-dessus.

4. Le 2,4-bis(4-chlorophényl)-3,3-diméthyl-1-(imidazol-1-yl)-2-butanol.

5. Le 3-(4-chlorophénoxy)-2-(4-chlorophényl)-3-méthyl-1-(1,2,4-triazol-1-yl)-2-butanol.

6. Le 2-(4-chlorophényl)-2-(4-chlorophényl-tert-butyl)-oxiranne.

7. Le 2-(4-chlorophényl)-2-[2-(p-chlorophénoxy)-prop-2-yl]-oxiranne.

8. Procédé de préparation d'hydroxyalkyl-azoles substitués de formule générale I de la revendication 1, caractérisé en ce qu'on fait réagir, en présence d'un diluant et éventuellement en présence d'une base, des oxirannes de formule générale II :

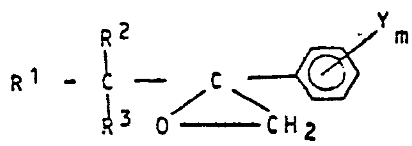

(II)

dans laquelle $R^1$, $R^2$, $R^3$, Y et m ont le sens indiqué ci-dessus, avec des azoles de formule générale III :

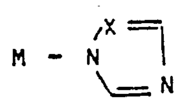

(III)

dans laquelle

X a le sens indiqué ci-dessus, et

M représente un atome d'hydrogène ou un métal alcalin, et éventuellement un oxyde de façon usuelle, selon les méthodes connues, les hydroxyalkyl-azoles ainsi obtenus, de formule générale Ia :

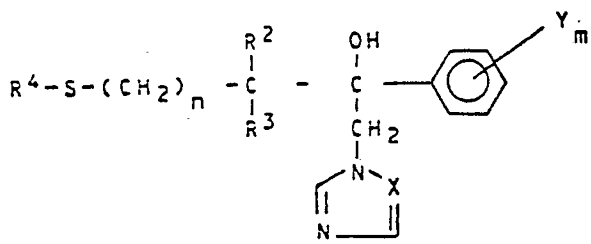

(Ia)

dans laquelle

$R^2$, $R^3$, X, Y et m ont le sens indiqué ci-dessus,

$R^4$ représente un groupe phényle éventuellement substitué par Y, et

n représente les nombres 0 ou 1.

9. Médicaments, caractérisés par une teneur en un hydroxyalkyl-azole substitué de formule générale I selon la revendication 1.